# EUROPEAN PATENT APPLICATION

(11) **EP 2 133 422 A1**
(43) Date of publication of application: **16.12.2009**
(21) Application number: 07715267.6
(22) Date of filing: 28.02.2007
(51) Int. Cl.: C12N 15/09, C12M 1/00, C12Q 1/68

(54) **BIOLOGICAL SUBSTANCE ANALYSIS CHIP, BIOLOGICAL SUBSTANCE ANALYSIS KIT AND METHOD OF BIOLOGICAL SUBSTANCE ANALYSIS USING THEM**

(71) Applicant: On-chip Cellomics Consortium, Tokyo 100-0006 (JP)
(72) Inventor: OKANO, Kazunori, Tokyo 160-0008 (JP); YASUDA, Kenji, Tokyo 135-0052 (JP)
(74) Representative: Prinz & Partner
(86) International application number: PCT/JP2007/054376
(87) International publication number: WO 2008/105105

(57) **Abstract**

The present invention provides a biological substance analysis chip capable of quantitative measurement in a minute region through immobilization of a biological substance trapping probe to a substrate surface in a more uniform density, a biological substance analysis kit that uses said chip, and a biological substance analysis method that uses the above.

## Description

### TECHNICAL FIELD

The present invention relates to a biological substance analysis chip for quantitative measurement of cells, mRNA (which is a gene product expressed in tissues that are cell aggregates) and proteins, a biological substance analysis kit, and a biological substance analysis method.

### BACKGROUND ART

Efforts to understand the living body at the DNA level, diagnose disease and comprehend life phenomena have accelerated as the Genome Project has progressed. It is useful to investigate gene expression status to understand life phenomena and investigate the workings of genes. Two methods of investigating gene expression status are investigating mRNA, a gene transcription product, and investigating the proteins translated from mRNA. A useful method of investigating mRNA is to use DNA probe arrays or DNA probe chips (Since derivatives of oligonucleotides are actually immobilized, these are also called oligo chips.) in which many DNA probes are divided by type and immobilized on a solid surface. In mRNA testing using DNA probe arrays, RNA components are extracted from an adequately large quantity of cellular specimens in advance, cDNA is synthesized through a reverse transcription reaction by reverse transcriptase, and amplification is carried out through PCR amplification and cRNA synthesis. Also, during complimentary strand synthesis, labeled dNTP is introduced and a large quantity of labeled DNA strands are obtained. Recently, a method wherein amino allyl dNTP is introduced and an amino group is reacted with fluorescent dye thereafter to obtain a fluorescent-labeled DNA strand has become the norm. The thusly obtained fluorescent-labeled DNA strand is hybridized with a so-called DNA probe array and various types of expressed gene analysis are performed. DNA probe arrays usually consist of minute spots from several dozen to 150µmϕ to which DNA probes are immobilized. When probes are immobilized in a high density, the rate at which labeled cDNA is trapped increases and highly-sensitive detection becomes possible.

Methods for producing DNA chips include utilizing a photochemical reaction and a method wherein lithography, which is often used in semiconductor engineering, is used to synthesize an array of oligomers laid out on a plurality of divided cells by single base (Science 251, 767-773 (1991)). In another method, DNA probes and protein probes are implanted one at a time in each chip section (Proc. Natl. Acad. Sci. USA 93, 4613-4918 (1996)).

These chips all have a structure wherein a plurality of probes are arranged in an array on the surface of a glass slide, etc. In general, only the physical position in which probes are immobilized provides indexing as to which probes are in which position. Although fluorescent dye is the substance predominantly used in the labeling of DNA chips during detection, electrochemical luminescence and detection methods wherein Redox (oxidation-reduction) reactions are used and detected as an electric signal are also in practical use.

In lieu of a DNA probe, the concept of a protein chip wherein an antibody or antigen is immobilized in an array on a substrate is also in practical use. An allergen detection system is in practical use for medical testing wherein a device to which a few dozen to a few hundred allergy-causing antigens (allergens) are immobilized is reacted with test serum atop a substrate, the IgM that causes the antigen-antibody reaction with each antigen and is present in the serum is specifically captured and reacted with the enzyme-labeled secondary antibody that bonds with IgM, and measurements of enzyme activity are performed by combining enzyme activity with chemiluminescence. In order to perform highly-accurate detection, the trapping rate for substances to be measured is also increased in protein chips by immobilizing an antibody or antigen to a solid surface at a high density.

When detecting a substance to be measured that was trapped atop a protein chip, there are many cases in which enzyme-labeled substances are used and the substance to be measured is detected with a chemiluminescent or bioluminescent system. In addition, there are many methods in practical use including fluorescent label detection, electrochemiluminescent detection, and cases in which analysis is performed with a mass spectrograph.

In the electrochemiluminescent method there is an antibody for antigen capture on the electrode surface and a ruthenium complex is used as the sandwich antibody labeling substance (Clin. Chem., 37, 1534-1539 (1991)). When ruthenium oxidation at the electrode surface is coupled with the redox reaction of the TPA and reduction occurs, the ruthenium electrons that entered the excited state return to the ground state and emit light.

As relates to the present invention, in the sandwich immunoassay method, which is a biological substance detection method and is a technology similar to the protein chip, it is reported that particles are used as a labeling substance and that the substance to be analyzed is subjected to molecular counting (Analytical Biochemistry 202, 120-125 (1992)).

### DISCLOSURE OF THE INVENTION

In the abovementioned related art, methods for the identification and detection of mRNA, which is a gene transcript product, or protein, which is a translation product, that uses a DNA probe chip or a protein chip have been investigated in detail, and measurements can be performed to an extent with some sort of method when solution samples could be prepared with some sort of method. In addition, as disclosed in patent application 2004-226361, patent application 2004-297194 or patent application 2004-318770, which were submitted by one of the applicants, methods in which nanoparticles are used to perform analysis have also been proposed in the mRNA and DNA microarray field.

However, since the affinity probes (Hereinafter the term affinity probe is a generic term applying to a substance used to bond to a biological substance in order to obtain some type of information useful in biological substance analysis.) used to trap the biological substance atop the substrate are a bioorganic substance such as a DNA probe or an antibody, immobilization to a glass or silicon substrate presents the following problems, namely:
1) Although physical adsorption and covalent bond formation are used to immobilize the affinity probes atop the substrate, both adsorption reactions and covalent bond reactions are dependent upon the state of the substrate surface. Accordingly, it is often difficult to achieve uniform immobilization.
2) The direction in which an affinity probe is immobilized to a substrate cannot be controlled three-dimensionally. In other words, it is difficult to point the affinity site for the trapping of the biological substance to be measured, which is in solution, to the position at which it will react with the biological substance to be measured. Consequently, the quantity of the biological substance to be measured that is trapped decreases due to a steric constraint of the affinity probe itself.
3) Affinity probe density cannot be controlled. Although it is generally thought that a substrate to which affinity probes of an arbitrary density are affixed can be obtained through control of affinity probe density during immobilization and duration of immobilization, actual control is often difficult. In the aforementioned background art (Analytical Biochemistry 202, 120-125 (1992)), the actual density of affinity probe immobilization is controlled through the introduction of a different biological substance while the affinity probes are immobilized. Attempting to immobilize a diluted affinity probe to a substrate surface results in an "island" of high density surrounded by a low density area.
4) Alternatively, in a problem related to 1) and 3) above, a reduction in the area of affinity probe immobilization will cause differences in the quantity of affinity probes immobilized in each area, possibly having a negative effect on quantitative measurement. Further, a reduction in the area of affinity probe immobilization will make it impossible to use a method such as fluorescence to measure the fluorescence intensity of the entire affinity probe area and quantitatively analyze the quantity of the target biological substance in the sample on the basis of the fluorescence intensity. Consequently, as in the aforementioned background art (Analytical Biochemistry 202, 120-125 (1992)) or inventions of the patent applications, a method is used wherein nanoparticles are bonded to the trapped target biological substance atop a substrate and said nanoparticles are then counted. However, if the affinity probes are not affixed uniformly, the number of nanoparticles bonded at each site will differ, obviously causing a decrease in the accuracy of quantitative detection.

There are optimum conditions for the density of affinity probes affixed to a substrate. Accordingly, the need to arbitrarily control affinity probe density arises.

In a conventional probe immobilization method often used, a silane coupling reaction was used to introduce a functional residue on the surface of glass or a silicon wafer, both of which are inorganic, and the affinity probes were then immobilized to said functional residue. The silane coupling reaction, however, was dependent upon the condition of the substrate surface and was not always an ideal method for uniform affinity probe immobilization.

The purpose of the present invention is to provide the following:
a biological substance analysis chip capable of performing quantitative measurement in a minute region by fixing biological substance trapping probes to a substrate surface in a more uniform manner;
a biological substance analysis kit that uses said chip; and
a biological substance analysis method that uses said chip and said kit.

Accordingly, the present invention provides the following biological substance analysis chip, biological substance analysis kit, and biological substance analysis method.
[1] A biological substance analysis chip characterized by a structure comprising a substrate and a solid chip having a trapping residue (third functional group) of a prescribed biological substance on a single surface of a substrate wherein:
   the substrate has a polymer layer prepared by polymerizing two types of monomer on a single surface of the substrate;
   the polymer layer has a structure wherein a first monomer having a first functional group in two locations and a second monomer having a second functional group for polymerizing in two locations are polymerized so as to be substantially arranged alternately;
      and either the first monomer or the second monomer contains the third functional group.
[2] A biological substance analysis chip according to Item 1 wherein the combination of the first monomer and the second monomer is a diisocyanate monomer having the structure NCS-R₁-NCS (where R₁ is an arbitrary residue) and a diamine monomer having the structure NH₂-R₂-NH₂ (where R₂ is an arbitrary residue) and the polymer layer is a polyurea.
[3] A biological substance analysis chip according to Item 1 or Item 2 wherein
   the combination of the first monomer and the second monomer is an acid anhydride having the structure R₁-C=O-O-O=C-R₂ or R₁-C=O-O-O=C-R₂-C=O-O-O=C-R₃ (wherein R₁ to R₃ are arbitrary residues) and a diamine monomer having the structure NH₂-R₅-NH₂ (wherein R₅ is an arbitrary residue) and the polymer layer is a polyamide or a polyimide.
[4] A biological substance analysis chip according to either Item 1 or Item 3 wherein the third functional group is a carboxyl group.
[5] A biological substance analysis chip according to Item 4 having a structure wherein isothiocyanic acid has been introduced into the carboxyl group.
[6] A biological substance analysis chip according to Item 4 or Item 5 having a structure wherein amine is introduced at the surface of the solid chip through reaction of the carboxyl group with a diamine and isothiocyanate residue is introduced at the surface of the solid chip through reaction of diisothiocyanate with the amine introduced at the surface of the solid chip.
[7] A biological substance analysis kit comprising:
   a solid chip having the trapping residue of the prescribed biological substance (third functional group), said solid chip having a polymer layer on its surface prepared through polymerization of two types of polymer wherein at least the polymer layer is a structure in which among the two polymerizing monomers, the first monomer, which has the first functional group in two locations, and the second monomer, which has the second polymerizing functional group in two locations, are substantially arranged alternately, at least one of the two monomers has a third functional group, and the molecule for trapping the prescribed biological substance (biological substance trapping probe) is immobilized through covalent bonding at the third functional group, wherein said chip is a cell composition analysis chip; and
   a biological substance comprising a labeling substance for specifying the prescribed biological substance bonded to the prescribed biological substance trapped in the molecule immobilized to the third functional group.
[8] A biological substance analysis kit according to Item 7 wherein the labeling substance comprises nanoparticles having a particle diameter of 5 to 300 nm.
[9] A biological substance analysis method comprising the following steps:
   adding a buffer including the biological substance to the space on the surface of a solid chip having the trapping residue of the prescribed biological substance (third functional group), said solid chip having a polymer layer on its surface prepared through polymerization of two types of polymer wherein at least the polymer layer is a structure in which among the two polymerizing monomers, the first monomer, which has the first functional group in two locations, and the second monomer, which has the second polymerizing functional group in two locations, are substantially arranged alternately, at least one of the monomers has a third functional group, and a molecule for trapping the prescribed substance (biological substance trapping probe) is immobilized through covalent bonding at the third functional group, wherein said chip is a cellular composition analysis chip;
   trapping the prescribed biological substance using the prescribed substance trapping molecule that is covalently bonded to and exists on the surface of the cellular composition analysis chip;
   bonding the biological substance labeled with the nanoparticles used to specify and bond to the prescribed biological substance; and
   counting the nanoparticles bonded to the surface of the cellular composition analysis chip.
[10] A biological substance analysis chip comprising a substrate having a biological substance trapping probe, and a polymer layer comprising a polymer comprised of a dimer unit formed from a first monomer and a second monomer on the surface of the substrate, wherein the biological substance trapping probe is bonded to the first monomer or the second monomer.
[11] A biological substance analysis chip according to Item 10 wherein the polymer layer comprises polyurea, polyamide, or polyimide.
[12] A biological substance analysis chip according to Item 10 wherein:
   the first monomer has two of the first functional group;
   the second monomer has two of the second functional group;
   the dimer is formed through bonding of a single first functional group of the first monomer to a single second functional group of the second monomer; and
   the polymer is formed through bonding of the first functional group and the second functional group of the dimer residue, each of which bonds to the second functional group or the first functional group of a different dimer.
[13] A biological substance analysis chip according to Item 12 wherein the first functional group is a isocyanato group and the second functional group is an amino group.
[14] A biological substance analysis chip according to Item 13 wherein the first monomer is a diisocyanate monomer having the structure NCS-R₁-NCS (where R₁ is an arbitrary residue) and the second monomer is a diamine monomer having the structure NH₂-R₂-NH₂ (where R₂ is an arbitrary residue).
[15] A biological substance analysis chip according to Item 10 wherein the first monomer is an acid anhydride having the structure R₁-C=O-O-O=C-R₂ or R₁-C=O-O-O=C-R₂-C=O-O-O=C-R₃ (where R₁ to R₃ is an arbitrary residue) and the second monomer is a diamine monomer having the structure NH₂-R₅-NH₂ (where R₅ is an arbitrary residue).
[16] A biological substance analysis chip described in any one of Items 10 to 15 wherein the first monomer or the second monomer further includes the third functional group and the biological substance trapping probes are bonded to the first monomer or the second monomer via the third functional group.
[17] A biological substance analysis chip according to Item 16 wherein the third functional group is a carboxyl group.
[18] A biological substance analysis chip according to Item 16 wherein the third functional group is an amino group.
[19] A biological substance analysis chip according to any one of Items 16 to 18 wherein the biological substance trapping probe contains a fourth functional group bonded thereto, and the biological substance trapping probe is bonded to either the first monomer or the second monomer due to bonding between the third functional group and the fourth functional group
[20] A biological substance analysis chip according to any one of Items 10 to 19 wherein the biological substance trapping probe is a polynucleotide, a polypeptide, or an antibody.
[21] A biological substance analysis chip according to any one of Items 10 to 20 wherein the biological substance analysis area comprising the polymer layer is formed in an array on the substrate.
[22] A biological substance analysis kit comprising the biological substance analysis chip according to any one of Items 10 to 21 and a labeling substance for labeling a biological substance bonded to the biological substance trapping probe.
[23] A biological substance analysis kit according to Item 22 wherein the labeling substance comprises gold nanoparticles having a particle diameter of 5 to 300 nm.
[24] A biological substance analysis method comprising the step of analyzing a biological substance through the use of the biological substance analysis chip according to any one of Items 10 to 21.
[25] A method according to Item 24 comprising:
   contacting the sample solution containing the biological substance with the polymer layer of the biological substance analysis chip described in any of Items 10 to 21;
   labeling the biological substance trapped in the polymer layer; and
   detecting or quantitating the labeled biological substance.

According to the present invention, a biological substance analysis chip wherein the biological substance probes are immobilized on a substrate in a uniform manner is provided. According to the present invention, the negative effects of a decrease in biological substance trapping proficiency due to a steric constraint of the biological substance trapping probe itself can be reduced as much as possible. According to the present invention, it is possible to control the biological substance trapping probe density by changing the types of monomers that comprise the polymer layer. According to the present invention, more accurate quantitative analysis of a biological substance in a test sample is possible.

### BRIEF DESCRIPTION OF THE DRAWINGS

Fig. 1 shows one embodiment of an optimum biological substance analysis chip for practicing the present invention. Fig.1 (A) is a two-dimensional diagram showing one example of the biological substance analysis chip of the present invention. Fig.1 (B) is a cross-section showing the A-A position in Fig.1 (A) as seen from the direction of the arrows.
Fig.2 (A) is a pattern diagram showing the cross section structure of polymer thin layer 21, wherein polymer thin layer 21 has alkyldiisocynate as its first monomer and diaminophenylacetic acid as its second monomer, is formed on the top surface of substrate 1 through evaporation polymerization, and wherein the first and second monomers were reacted in an equimolar ratio and then polymerized. Fig.2 (B) is a pattern diagram showing the planar structure of polymer thin layer 21.
Fig.3 is a diagram showing a pattern depicting the bond between the third functional group 26 in polymer thin layer 21 formed on the surface of substrate 1 and functional group 33 of 5' terminal portion 32 of DNA probe 31.
Fig.4 (A) to Fig.4 (D) are diagrams showing an mRNA detection process using the substrate shown in Fig.1 to Fig.3.
Fig.5 (A) to Fig.5 (D) are diagrams showing an mRNA detection process wherein multiple DNA probes are immobilized to an identical spot position and a DNA microarray having a different DNA probe is used at each spot position.

### BEST MODE FOR CARRYING OUT THE INVENTION

One embodiment of the present invention provides a biological substance analysis chip comprising a substrate having a biological substance trapping probe. Said biological substance analysis chip has, on the surface of the substrate, a polymer layer comprising a polymer comprising a dimer unit formed from the first monomer and the second monomer. The biological substance trapping probe is bonded to the first monomer or the second monomer.

As used in the present specification,"biological substance" means material to be tested in biological samples such as polynucleotides, for example DNA and RNA (i.e. mRNA, a gene transcription product), proteins or peptides, which are a product of DNA translation, and antigens and antibodies.

As used in the present specification, "polymer" means a polymer of a chemical substance used to form the polymer layer whose purpose is the trapping of biological substances. Polymers used in the present invention are heteropolymers formed from polymerization of a dimer formed from the first monomer and the second monomer as the smallest repeating unit.

As used in the present specification, a "biological substance trapping probe" can, according to biological substance type, be a polynucleotide, polypeptide, antigen, antibody, etc. that functions as a bonding means for trapping the biological substance at the polymer layer. For example, if the biological substance is an mRNA, a polynucleotide having a complimentary sequence that can hybridize with the mRNA can be used as the biological substance trapping probe. Alternatively, if the biological substance is an antigen, an antibody that bonds specifically thereto can be used as the biological substance trapping probe. Typically, the biological substance probe can bond to the polymer layer through bonding with the first monomer or the second monomer in the polymer, for example, through an anchor means such as a specified functional group.

In the present invention, silane coupling, which would react directly with residues such as Si-OH or SI=O on a glass or silicon substrate surface or Me-OH or Me=O (Me: metallic atom) on a metal surface, is not used. The quantity of -OH and =O residues on these types of surfaces varies in accordance with surface status. Consequently, the uniform introduction of an active group was thought to be intrinsically difficult. Therefore, it was necessary to introduce a residue that would fix the biological substance trapping probes regardless of substrate surface condition. To facilitate this introduction, the polymer layer must be introduced onto the substrate surface regardless of substrate surface condition, and said polymer layer should be designed so that it has a functional group (third functional group) such that the biological substance trapping probes are immobilized at regular intervals. When performing the above, it is crucial to align the intervals of the third functional group used to fix the biological substance trapping probes.

In the present invention, two types of monomer are polymerized, resulting in a product having a polymerization thin film made from the prepared polymer layer. The polymer layer has a polymerized structure wherein the first monomer, which has the first polymerization functional group in two locations, and the second monomer, which has the second polymerization functional group in two locations, are arranged alternately and either the first monomer or the second monomer has a biological substance trapping probe bonding residue (third functional group). Specifically, diisocyanates as the first monomer and diamines as the second monomer are reacted in an equimolar ratio on the substrate and polymerized. In this case, the first functional group will be an isocyanate group and the second group will be an amino group. Examples of diisocyanate that can be used are alkyl diisocyanates and phenylene diisocyanates. Monomers in which the third functional group has been introduced into the side chain of diisocyanates or diamines can be used for biological substance trapping probe bonding.

For example, a monomer is used in which a carboxyl group has been introduced into the side chain of the diamines as the third functional group. When this monomer is polymerized on the substrate surface, a polymer in which diisocyanates and diamines are alternately bonded is obtained. Accordingly, the carboxyl group which is the third functional group will exist in the diamine part of the side chain of the dimer unit consisting of diisocyanates and diamines. Consequently, the biological substance trapping probes can be immobilized in a regular interval on top of the polymer via the third functional group. The polymerization product thin layer in this case is polyurea. Regarding polymerization conditions, the interval of the carboxyl group to be introduced can be adjusted by adjusting the length of the main chain of diisocyanates and diamines that can be used in preexisting vacuum deposition methods. Further, by so doing, thin films having a functional group can be freely formed even on electrodes of gold, silver, etc. onto whose surfaces it was conventionally difficult to introduce functional groups (third functional group).

Using a carbodiimide system activator, a biological substance trapping probe having an amino group (fourth functional group) can be directly immobilized to a carboxyl group introduced atop the substrate. Alternatively, if ethylenediamine is reacted with this side chain carboxyl group in the presence of a dicyclohexylcarbodiimide system activator, a primary amine residue can be introduced into a side chain carboxyl group at a high recovery rate. In other words, the primary amines will be lined up in an approximately regular interval on the substrate surface. Next, the primary amines are modified with a 1, 4-phenylene diisothiocyanate described in Nucleic Acids Research, 27, 1970-1977 (1999) and CHEMBIOCHEM 2, p. 686-694 (2001), and an isothiocyanate group is thereby introduced at the surface of the substrate. Finally, a DNA probe (spacer part: 5'-terminal side 10 bases + part that reacts with the target cDNA: average 32 bases in length) having an amino group (fourth functional group) is reacted at the 5'-terminal. This reaction condition is detailed in the above-mentioned documents. Using the above-mentioned method, a substrate to which DNA probes are immobilized in an approximate 9 nm interval can be obtained. The term "third functional group" used in the present invention is thereby not limited to a residue comprising a single type of residue, but also means a reactive residue formed from the bonding or combination of multiple types of residue.

In addition to the example in which polyurea having a carboxyl group is formed on a substrate, any polymer can also be used if said polymer periodically has a residue into which can be introduced a reaction group that can immobilize polynucleotides, peptides, etc. which are probes in a side chain. For example, a combination of two types of monomer can be used in which the monomers are an acid anhydride having the structure R₁-C=O-O-O=C-R₂ or R₁-C=O-O-O=C-R₂-C=O-O-O=C-R₃ (R₁ to R₃ are arbitrary residues) and a diamine monomer having the structure NH₂-Rs-NH₂ (R₅ is an arbitrary residue) where R₅ is a diaminophenylacetate monomer having a carboxyl group. An acid anhydride with a structure such as 4,4'-(hexafluoroisopropylidene)-bis-(phthalic anhydride) can be used.

This type of polymer is, namely, a polyimide. Generally used polyimides are this sort of bis(phthalic anhydride) and bis(amino) compound. Alternatively, for example, a diaminophenylacetate having a long carboxyl group or a monomer having a carboxylate residue in the aliphatic position of an aliphatic diamine such as 1, 10-diaminodecane and a polyamide that forms a film on a substrate through evaporation polymerization of terephthaloyl dichloride, a diacidchloride monomer, can be used. Various reactive groups are introduced into a long chain aliphatic diamine for the purpose of probe immobilization. It is sufficient if evaporation polymerization is carried out according to preexisting methods, for example the methods described in Jpn. J. Appl. Phys. 33, L1721-L1724 (1994) and Thin Solid Films, 215, 94-97 (1992), and in consideration of the vaporization conditions of the monomer in use.

Hereinbelow, preferred examples of the present invention are described with reference to the drawings.

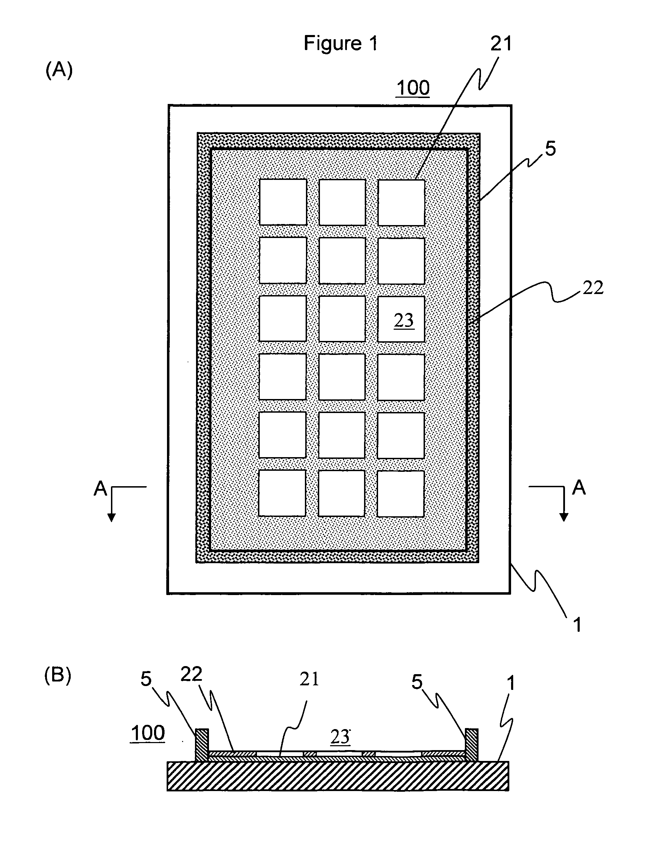

Fig. 1 shows an example of a preferred biological substance analysis chip for the practice of the present invention.

Fig. 1(A) is a two-dimensional diagram showing a biological substance analysis chip in one embodiment of the present invention.

Fig. 1(B) is a cross-section seen from the direction of the arrows in the A-A position in Fig. 1(A).

This biological substance analysis chip 100 forms polymer thin film layer 21 for immobilizing affinity functional groups on the upper surface of glass substrate 1. Teflon (registered trademark) layer 22 is printed on polymer layer 21. Consequently, polymer layer 21, which appears on the surface, is in sections such as 23 in the Figure. These sections are 50×50µm and can store one cell. polyT (T30) is immobilized on polymer thin film 21 as a biological substance trapping probe, thereby enabling comprehensive trapping of mRNA. Alternatively, a chip for trapping specific mRNA or cDNA can be obtained by immobilization of the DNA probes described in Disclosure of the Invention above (spacer part: 5'terminal side 10 bases + part that reacts with the target mRNA or cDNA: average 32 bases in length). This will, of course, result in a structure spotted with a plurality of DNA probes like that of an ordinary DNA chip. Divider 5 exists as necessary in the vicinity of polymer layer 21 to prevent the outflow of sample solution to the surrounding area. Divider 5 can be made of resin or the like.

First, a polymer thin film layer for immobilization of affinity functional groups that form on the top surface of substrate 1 will be explained. Polymer thin film layer 21 reacts to polymerize an alkyl diisocyanate as the first monomer and a diaminophenylacetate as the second monomer in an equimolar ratio. In other words, a urea resin thin film is the polymer thin film. Hexamethylene diisocyanates, for example, can be used as an alkyl diisocyanate. In addition, the third functional group is the carboxyl group of diaminophenylacetate, which is the second monomer. Regarding polymerization conditions, a preexisting vacuum deposition method can be used.

The structure of polyurea thin film 21 thereby formed on the substrate has a structure formed through alternate reaction of alkyl diisocyanates and diaminophenyl acetate monomers. Accordingly, the calboxylic acid groups will exist in a comparatively uniform interval. The interval of the carboxyl group to be introduced can be adjusted by adjusting the length of the alkyl group. In addition, by so doing, thin films having a functional group can be formed even on electrodes of gold, silver, etc. onto whose surfaces it was conventionally difficult to introduce functional groups. Using a carbodiimide system activator, a probe having an amino group as its third functional group can be directly immobilized to a carboxyl group introduced atop the substrate. Alternatively, if ethylenediamine is reacted with this side chain carboxyl group in the presence of a dicyclohexylcarbodiimide system activator, a primary amine residue can be introduced into a side chain carboxyl group at a high recovery rate. In other words, the primary amines will be lined up in an approximately regular interval on the substrate surface.

Next, the primary amines are modified with a 1,4-phenylene diisothiocyanate described in Nucleic Acids Research, 27, 1970-1977 (1999) and CHEMBIOCHEM 2, p.686-694 (2001) and an isothiocyanate group is thereby introduced on the surface of the substrate. Finally, the primary amines are reacted with polyT (T30) having an amino group at its 5'terminal. This reaction condition is detailed in the above-mentioned documents. Using the above-mentioned method, a substrate to which polyT is immobilized in an approximate 9 nm interval can be obtained.

Although a polyurea having a carboxyl group was formed and used on substrate 1 in the present embodiment, a polymer periodically having a residue capable of introducing a reactive group that can immobilize a polynucleotide or a peptide, etc. as a probe in the side chain can also be used. For example, a combination of two types of monomer wherein the monomers are an acid anhydride having the structure R₁-C=O-O-O=C-R₂-R₃-C=O-O-O=C-R₄ (R₁ to R₄ are arbitrary residues) and a diamine monomer having the structure NH₂-R₅-NH₂ (R₅ is an arbitrary residue) where R₅ is a diaminophenylacetate monomer having a carboxyl group can be used. An acid anhydride with a structure such as 4,4'-(hexafluoroisopropylidene)-bis-(phthalic anhydride) can be used. This type of polymer is, namely, a polyimide. Generally used polyamides are this sort of bis(phthalic anhydride) and bis(amino) compound. Alternatively, for example, a diaminophenylacetate having a carboxyl group, or a monomer having a carboxylate residue in the aliphatic position of an aliphatic diamine such as 1,10-diaminodecane and terephthaloyl chloride, a dichloride monomer, are subjected to evaporation polymerization, resulting in a polyamide that forms a film on a substrate, can be used. It is sufficient if various reactive groups are introduced into a long chain aliphatic diamine for the purpose of probe immobilization. It is sufficient if evaporation polymerization is carried out according to preexisting methods such as the methods described in Jpn. J. Appl. Phys. 33, L1721-L1724 (1994) and Thin Solid Films, 215, 94-97 (1992), and in consideration of the vaporization conditions of the monomer in use.

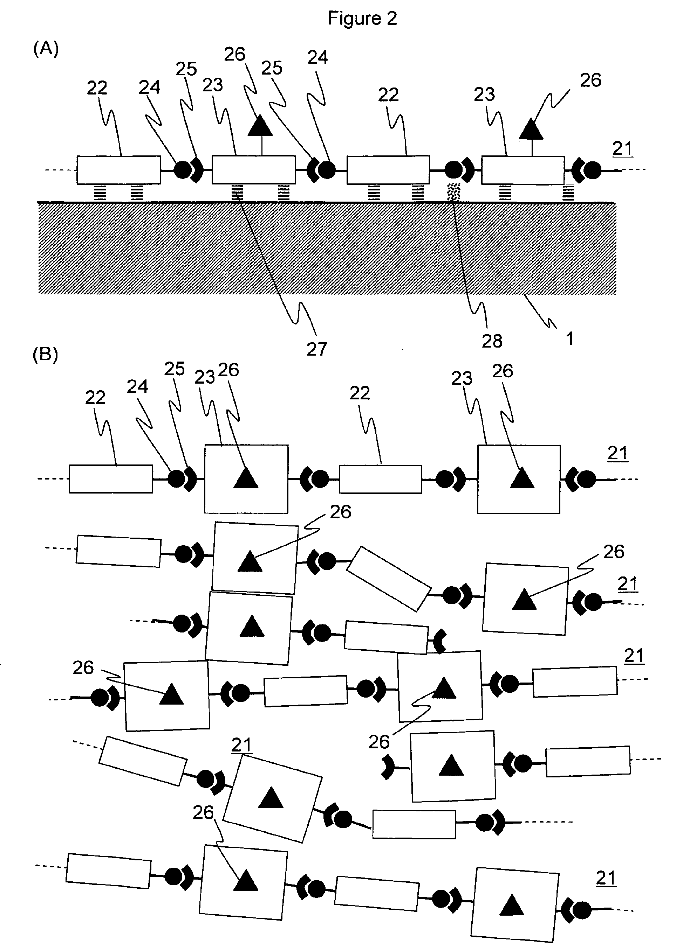

Fig. 2(A) is a pattern diagram showing the cross-sectional structure of polymer thin film layer 21, which is formed by evaporation polymerization on the upper surface of substrate 1 when alkyl diisocyanate as the first monomer and diaminophenylacetate as the second monomer are reacted in an equimolar ratio and polymerized. Fig. 2(B) is a pattern diagram showing the planar configuration of polymer thin film layer 21.

Polymer thin film layer 21 is a chain architecture of two alternately polymerized monomers in which first monomer part 22 and second monomer part 23 are linked to each other such that first functional group 24 of monomer part 22 and second functional group 25 of second monomer part 23 are linked to each other. Third functional group 26 exists in second monomer part 23 as a side chain. It is supposed that polymer layer thin film 21 is immobilized to the substrate surface by weak bond 27 and covalent bond state 28, which exists on a small section of the substrate between a silanol group on the glass surface of substrate 1 and pi electrons of the functional group on the polymer side. As can be understood by referring to Fig. 2(B), the first functional group 24 of the first monomer part 22 and the second functional group 25 of the second monomer part 23 are linked to each other. Also, since not all chain architectures of two alternately polymerized monomers line up in a perfect parallel array, third functional group 26 is not perfectly aligned in a uniform manner. In other words, as shown in Fig. 2(B), reversed and tilted monomer parts are intermixed. However, since second monomer part 23 having third functional group 26 is arranged in between first monomer part 22 and another first monomer part 22, excessive aggregation and sparse distribution of third functional group 26 can be prevented.

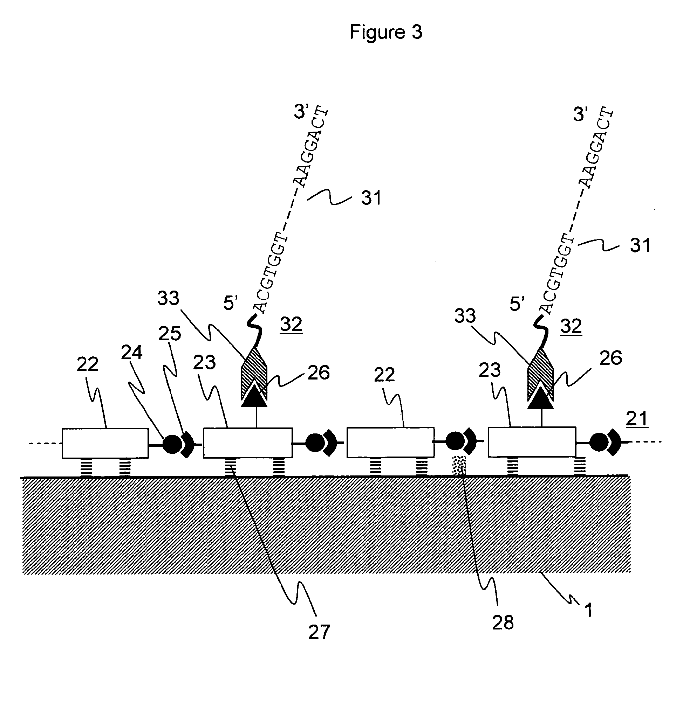

Fig. 3 is a pattern diagram showing a bond between third functional group 26 of polymer thin film 21 formed on the surface of substrate 1 and fourth functional group 33 of 5'terminal part 32 of DNA probe 31. Functional group 33 is composed of an amino group, a carboxyl group, etc. For example, when functional group 26 is a carboxyl group, an amino group can be used as functional group 33. Alternatively, for example, when functional group 26 is an amino group, a carboxyl group can be used as functional group 33. Combinations of the third functional group and the fourth functional group are not limited to those shown here. Here, regarding polymer thin film layer 21, since first monomer 22 and second monomer 23 are bonded to each other and polymerized, the interval of third functional group 26, which is a side chain of second monomer 23, is almost uniform when seen at the molecular level. Consequently, DNA probe 31 also bonds to third functional group 26 at an almost uniform interval. Although two-dimensional spread between polymer chains must also be considered as a matter of course, the gap between different chains can be expected to be largely uniform as shown in Fig. 2(B) due to the minus charge of the repulsion force of the carboxyl group of the side chain (corresponds to third functional group 26 in Fig. 2(A)).

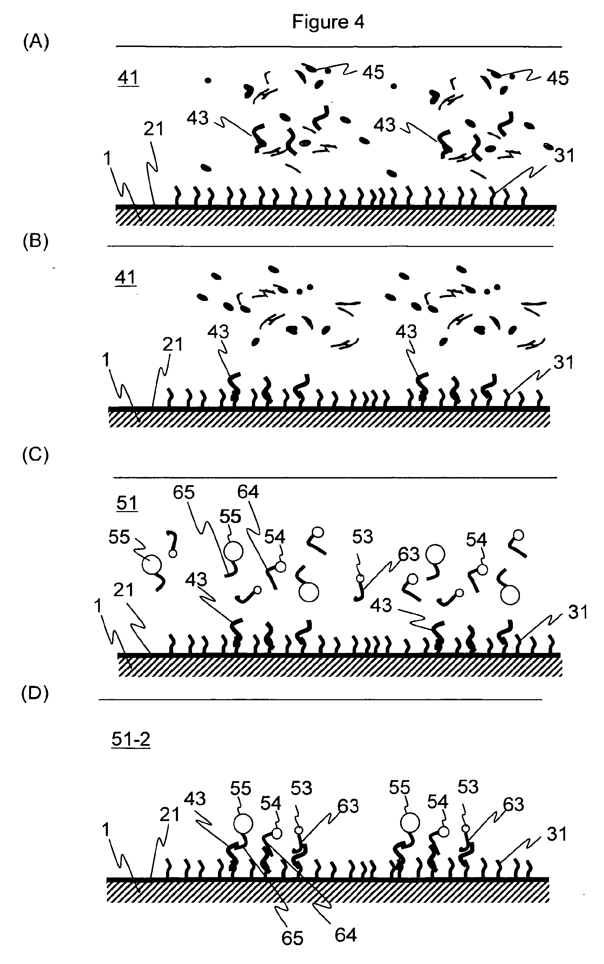

Fig. 4 is a figure showing an mRNA detection step using the substrate shown in Figs 1 to 3. Fig. 4(A) is a cross-section schematically showing, for example, a case in which mRNA 43 and protein 45 are dispersed in sample solution 41, which is obtained by cell fracture, and arranged on the upper surface of polymer thin film layer 21, which is located on the upper surface of substrate 1. A so-called PBS that does not contain EDTA is used as solution 41. Probe 31 1 is immobilized to the surface of polymer thin film layer 21 on the upper surface of substrate 1. Here, a derivation of polyT is used as probe 31. Since mRNA is being measured, cell permeable RNase inhibitor is inserted into the liquid droplets in advance to minimize mRNA degradation.

Fig. 4(B) is a diagram schematically showing the status of Fig. 4(A) after the passage of time. As time passes, mRNA 43 hybridizes to poly T probe 31 on the substrate. Trapping to the poly A probe of the substrate surface due to mRNA hybridization requires approximately 4 hours.

Fig. 4(C) explains the method for identifying mRNA group 43 trapped in polyT probe 31 on the upper surface of the thin polymer layer 21 on the upper surface of substrate 1. First, substrate 1 having a mRNA group trapped in polyT probe 31 shown in Fig. 4(B) is washed with 50 mM phosphate buffer with a pH of 7.4 containing 0.5% SDS and 0.1 M of NaCl. Next, since there are many types of mRNA group 43 which was hybridized with polyT probe 31, mixed solution 51 of the labeled probe is added in order to identify said types. Here, gold nanoparticles of different particle diameters to which DNA probes having different DNA sequences are bonded are used as labeled probes. The figure schematically shows a substrate to which solution 51 containing the following 3 types of DNA probe have been added: DNA probe 63, which is labeled with gold nanoparticle 53 having a particle diameter of 5 nm; DNA probe 64, which is labeled with gold nanoparticle 54 having a particle diameter of 10 nm; and DNA probe 65, which is labeled with gold nanoparticle 55 having a particle diameter of 15 nm. In order to prevent nonspecific adsorption of the gold nanoparticle-labeled DNA probe, solution 51 is a composition of 50 mM phosphate buffer with a pH of 7.4 containing 0.5% SDS and 0.1 M of NaCl.

After incubation for 1 hour at 45°C, washing is performed using the same buffer, and then washing is performed using a pH 7 citrate buffer solution containing 10mM of NaCl. In this state, a triple bond exists on the substrate wherein an mRNA group is hybridized to polyT probe 31, and labeled probes 63, 64, and 65 are hybridized to the mRNA, which is trapped in polyT probe 31. Gold nanoparticle-labeling substances 53, 54, and 55, which have different particle diameters and can be easily identified with a scanning electron microscope or a scanning atomic force microscope, are bound to labeled probes 63, 64, and 65. Accordingly, by counting gold nanoparticles 53, 54, and 55 while distinguishing particle diameter, the type and quantity of mRNA trapped in polyT probe 31 on substrate 1 can be measured.

Since sections 21 are minute, measuring only 5×5µm, the number of probes that can be immobilized to said sections is limited. If an attempt is made to maintain a constant immobilized probe quantity for each section, it will not be possible to use a method whereby the immobilization quantity depends on the condition of the substrate surface. Through use of said method, the space between probe molecules on the substrate surface can be maintained at approximately 9 nm. Consequently, through the present invention, the variation in the immobilized probe quantity for an approximate 5×5µm spot can be reduced from the approximate CV=30% of conventional methods to approximately CV=7%.

Next, the identification capabilities will be explained using an example of the present invention.

Although probe 31 is immobilized to the surface of polymer thin film layer 21 on the upper surface of substrate 1, an example is considered in which polyT is used as probe 31 and, as in an ordinary DNA chip, multiple DNA probe strands are "spotted" onto the surface of polymer thin film layer 21 and used.

In other words, an inkjet device is used to place a spot of each probe on a surface so that isothiocyanate residue prepared as above is arranged at an almost uniform interval. Spot size is 1 µmϕ, and each spot is lined up at a 2µm pitch.

Further, 3 types of capture DNA probe are immobilized as a mixture in each spot and, as explained above, detection DNA probes labeled by 3 types of gold nanoparticles with different particle diameters are used as detection DNA probes that correspond to capture probes.

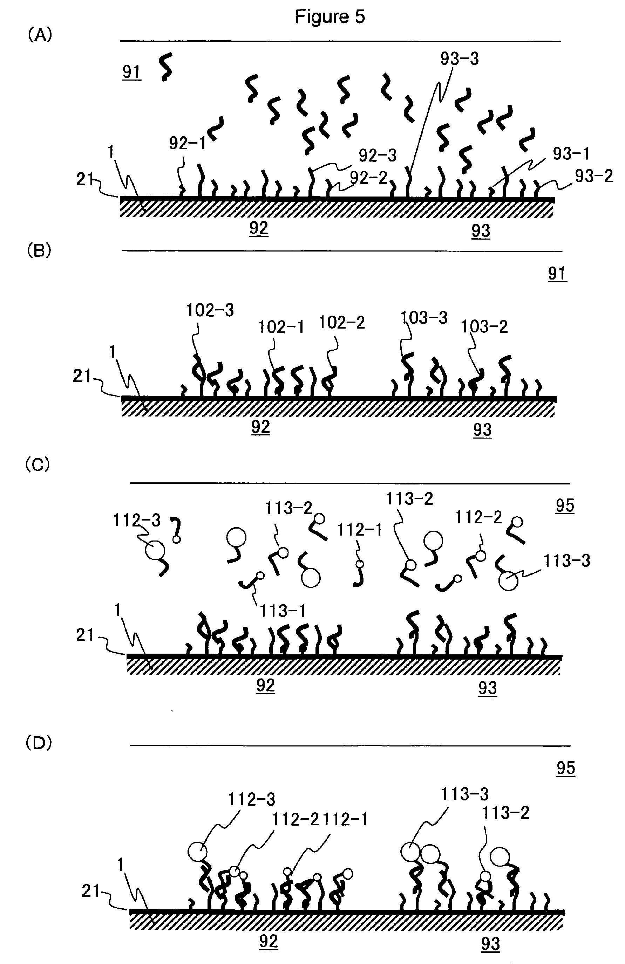

Fig. 5(A) shows a cross-section of a DNA microarray example in which three types of DNA probe that use the present invention are immobilized to the same spot position and a different DNA probe is immobilized to each spot. A group of the three probe types 92-1, 92-2, and 92-3 and three different probe types 93-1, 93-2, and 93-3 are individually immobilized to spot areas 92 and 93 on top of substrate 1 through the use of an active group of layer 21, on which a reactive residue produced through evaporation polymerization is lined up in an almost uniform interval. Sample mixed liquid 91 is added and incubation is performed for 2 hours at 50°C. Sample mixed liquid 91 is a single-chain cDNA mixture and is obtained by, for example, reverse transcription of a white blood cell-derived mRNA mixture. The solvent is 50 mM phosphate buffer (pH 7.4) containing 0.5% SDS. Following completion of the reaction, washing is performed with 50 mM phosphate buffer (pH 7.4) containing 0.5% SDS.

Fig. 5(B) shows a state in which cDNA has been trapped by probes on the surface of layer 21 on top of a substrate. Disparate cDNA 102-1, 102-2 and 102-3 hybridize to corresponding DNA probes in spot area 92, and cDNA 103-2 and 103-3 hybridize to corresponding DNA probes in spot area 93. The sample has no instances of cDNA that corresponds to probe 93-1 of spot 93. Accordingly, no evidence of hybridization is observed in any spot area.

Fig. 5(C) shows the state of a substrate wherein phosphate buffer 95 has been added to cause a reaction between the cDNA captured by the probes on the surface of layer 21 on the substrate and the second DNA probes 112-1, 112-2, 112-3, 113-1, 113-2, and 112-3, which are labeled with three types of gold nanoparticles having a particle size of 5, 10, and 15 µm. The second DNA probe has a complimentary sequence to each single strand cDNA 102- 1, 102- 2, 102- 3, 103- 1, 103- 2, and 103- 3 (cDNA 103-1 was not included in the sample and therefore does not appear in the drawing.). The reaction is carried out at 55°C for 30 minutes in 50 mM of phosphate buffer (pH 7.4) 95 containing 0.5% SDS. The gold nanoparticle-labeled DNA probe mixture is preheated at 90°C for 5 seconds, chilled to 55°C, and then immediately dripped onto the substrate. The substrate is then washed with 50 mM of phosphate buffer (pH 7.4) containing 0.5% SDS.

Fig. 5(D) shows a state in which DNA probes labeled with gold nanoparticles 112-1, 112-2, 112-3, 113-2, and 113-3 are hybridized to each spot area on the substrate. The substrate surface is scanned with a scanning electron microscope or scanning atomic force microscope and the gold nanoparticles bonded to each spot area are counted on the basis of particle diameter.

Since sections 21 are minute, measuring only 5×5µm, the number of probes that can immobilize to said sections is limited. If an attempt is made to maintain a constant immobilized probe quantity for each section, fluctuations in the dispersion of the immobilized quantity will become more pronounced. Through use of this method, the space between probe molecules on the substrate surface can be maintained at approximately 9 nm. When three types of probe are immobilized, the variation in the probe immobilization quantity for an approximate 5×5µm spot can be reduced from the approximate CV=40% of conventional methods to approximately CV=10%.

Although the above explains the illustrative examples of the present invention, various changes, modifications, and improvements could easily be arrived at by one skilled in the art. The above merely illustrates the principles of the present invention. One skilled in the art could implement a variety of changes without departing from the scope and spirit of the present invention.

### Industrial Applicability

The present invention will make it possible to detect intracellularly expressed mRNA and proteins at the molecular level. The present invention is useful as a biological substance analysis chip for the quantitative measurement of mRNA and proteins, which are a gene product expressed in cells and tissues that are cell aggregates, a biological substance analysis kit and a biological substance analysis method.

## Claims

1. A biological substance analysis chip **characterized by** a structure comprising a substrate and a solid chip having a trapping residue (third functional group) of a prescribed biological substance on a single surface of a substrate wherein:
the substrate has a polymer layer prepared by polymerizing two types of monomer on a single surface of the substrate;
the polymer layer has a structure wherein a first monomer having a first functional group in two locations and a second monomer having a second functional group for polymerizing in two locations are polymerized so as to be substantially arranged alternately;
and either the first monomer or the second monomer contains the third functional group.

2. A biological substance analysis chip according to Claim 1 wherein the combination of the first monomer and the second monomer is a diisocyanate monomer having the structure NcS-R₁-NCS (where R₁ is an arbitrary residue) and a diamine monomer having the structure NH₂-R₂-NH₂ (where R₂is an arbitrary residue) and the polymer layer is a polyurea.

3. A biological substance analysis chip according to Claim 1 or Claim 2 wherein
the combination of the first monomer and the second monomer is an acid anhydride having the structure R₁-C=O-O-O=C-R₂ or R₁-C=O-O-O=C-R₂-C=O-O-O=C-R₃ (wherein R₁ to R₃ are arbitrary residues) and a diamine monomer having the structure NH₂-R₅-NH₂ (wherein R₅ is an arbitrary residue) and the polymer layer is a polyamide or a polyimide.

4. A biological substance analysis chip according to either Claim 1 or Claim 3 wherein the third functional group is a carboxyl group.

5. A biological substance analysis chip according to Claim 4 having a structure wherein isothiocyanic acid has been introduced into the carboxyl group.

6. A biological substance analysis chip according to Claim 4 or Claim 5 having a structure wherein amine is introduced at the surface of the solid chip through reaction of the carboxyl group with a diamine and isothiocyanate residue is introduced at the surface of the solid chip through reaction of diisothiocyanate with the amine introduced at the surface of the solid chip.

7. A biological substance analysis kit comprising:
a solid chip having the trapping residue of the prescribed biological substance (third functional group), said solid chip having a polymer layer on its surface prepared through polymerization of two types of polymer wherein at least the polymer layer is a structure in which among the two polymerizing monomers, the first monomer, which has the first functional group in two locations, and the second monomer, which has the second polymerizing functional group in two locations, are substantially arranged alternately, at least one of the two monomers has a third functional group, and the molecule for trapping the prescribed biological substance (biological substance trapping probe) is immobilized through covalent bonding at the third functional group, wherein said chip is a cell composition analysis chip; and
a biological substance comprising a labeling substance for specifying the prescribed biological substance bonded to the prescribed biological substance trapped in the molecule immobilized to the third functional group.

8. A biological substance analysis kit according to Claim 7 wherein the labeling substance comprises nanoparticles having a particle diameter of 5 to 300 nm.

9. A biological substance analysis method comprising the following steps:
adding a buffer including the biological substance to the space on the surface of a solid chip having the trapping residue of the prescribed biological substance (third functional group), said solid chip having a polymer layer on its surface prepared through polymerization of two types of polymer wherein at least the polymer layer is a structure in which among the two polymerizing monomers, the first monomer, which has the first functional group in two locations, and the second monomer, which has the second polymerizing functional group in two locations, are substantially arranged alternately, at least one of the monomers has a third functional group, and a molecule for trapping the prescribed substance (biological substance trapping probe) is immobilized through covalent bonding at the third functional group, wherein said chip is a cellular composition analysis chip;
trapping the prescribed biological substance using the prescribed biological substance trapping molecule that is covalently bonded to and exists on the surface of the cellular composition analysis chip;
bonding the biological substance labeled with the nanoparticles used to specify and bond to the prescribed substance; and
counting the nanoparticles bonded to the surface of the cellular composition analysis chip.

10. A biological substance analysis chip comprising a substrate having the biological substance trapping probe, and a polymer layer comprising a polymer comprised of a dimer unit formed from a first monomer and a second monomer on the surface of the substrate, wherein the biological substance trapping probe is bonded to the first monomer or the second monomer.

11. A biological substance analysis chip according to Claim 10 wherein the polymer layer comprises polyurea, polyamide, or polyimide.

12. A biological substance analysis chip according to Claim 10 wherein:
the first monomer has two of the first functional group;
the second monomer has two of the second functional group;
the dimer is formed through bonding of a single first functional group of the first monomer to a single second functional group of the second monomer; and
the polymer is formed through bonding of the first functional group and the second functional group of the dimer residue, each of which bonds to the second functional group or the first functional group of a different dimer.

13. A biological substance analysis chip according to Claim 12 wherein the first functional group is a isocyanato group and the second functional group is an amino group.

14. A biological substance analysis chip according to Claim 13 wherein the first monomer is a diisocyanate monomer having the structure NCS-R₁-NCS (where R₁ is an arbitrary residue) and the second monomer is a diamine monomer having the structure NH₂-R₂-NH₂ (where R₂ is an arbitrary residue).

15. A biological substance analysis chip according to Claim 10 wherein the first monomer is an acid anhydride having the structure R₁-C=O-O-O=C-R₂ or R₁-C=O-O-O=C-R₂-C=O-O-O=C-R₃ (where R₁ to R₃ is an arbitrary residue) and the second monomer is a diamine monomer having the structure NH₂-R₅-NH₂ (where R₅ is an arbitrary residue).

16. A biological substance analysis chip described in any one of Claims 10 to 15 wherein the first monomer or the second monomer further includes the third functional group and the biological substance trapping probes are bonded to the first monomer or the second monomer via the third functional group.

17. A biological substance analysis chip according to Claim 16 wherein the third functional group is a carboxyl group.

18. A biological substance analysis chip according to Claim 16 wherein the third functional group is an amino group.

19. A biological substance analysis chip according to any one of Claims 16 to 18 wherein the biological substance trapping probe contains a fourth functional group bonded thereto, and the biological substance trapping probe is bonded to either the first monomer or the second monomer due to bonding between the third functional group and the fourth functional group

20. A biological substance analysis chip according to any one of Claims 10 to 19 wherein the biological substance trapping probe is a polynucleotide, a polypeptide, or an antibody.

21. A biological substance analysis chip according to any one of Claims 10 to 20 wherein the biological substance analysis area comprising the polymer layer is formed in an array on the substrate.

22. A biological substance analysis kit comprising the biological substance analysis chip according to any one of Claims 10 to 21 and a labeling substance for labeling a biological substance bonded to the biological substance trapping probe.

23. A biological substance analysis kit according to Claim 22 wherein the labeling substance comprises gold nanoparticles having a particle diameter of 5 to 300 nm.

24. A biological substance analysis method comprising the step of analyzing a biological substance through the use of the biological substance analysis chip according to any one of Claims 10 to 21.

25. A method according to Claim 24 comprising:
contacting the testing solution containing the biological substance with the polymer layer of the biological substance analysis chip described in any of Claims 10 to 21;
labeling the biological substance trapped in the polymer layer; and
detecting or quantitating the labeled biological substance.
